# EUROPÄISCHE PATENTSCHRIFT

(11) **EP 1 443 873 B1**
(45) Veröffentlichungstag und Bekanntmachung des Hinweises auf die Patenterteilung: **28.03.2007**
(21) Anmeldenummer: 02790356.6
(22) Anmeldetag: 12.11.2002
(51) Int. Cl.: A61F 2/01, C22F 1/00, A61B 6/00, A61F 2/88, A61F 2/84

(54) **MEDIZINISCHES IMPLANTAT**
MEDICAL IMPLANT
IMPLANT MEDICAL

(30) Priorität: 12.11.2001 DE 10155191
(43) Veröffentlichungstag der Anmeldung: 11.08.2004
(73) Patentinhaber: Dendron GmbH, 44799 Bochum (DE)
(72) Erfinder: HENKES, Hans, 44807 Bochum (DE); MONSTADT, Hermann, 44797 Bochum (DE); SPEDER, Jürgen, 44807 Bochum (DE); KONTEK, Ronald, 44651 Herne (DE)
(74) Vertreter: Thiel, Christian
(86) Internationale Anmeldenummer: PCT/EP2002/012617
(87) Internationale Veröffentlichungsnummer: WO 2003/041615

(56) Entgegenhaltungen:
- WO-A-02/03889
- WO-A-92/22263
- WO-A-95/18585
- WO-A-98/33454
- US-A- 5 108 407
- US-A- 5 637 089

## Beschreibung

Die Erfindung betrifft ein medizinisches Implantat in Form mindestens eines langgestreckten Filamentes, wobei das Filament zur Einnahme einer übergeordneten Struktur vorgeformt ist, die es am Implantationsort während der Implantation einnimmt.

Die Erfindung betrifft weiterhin eine Vorrichtung zur Implantation solcher Implantate in Körperhöhlen und -gefäßen.

Es ist bekannt, Gefäßverengungen (Stenosen) durch den Einsatz von Stents (Gefäßendoprothesen, Gefäßstützen) zu behandeln, welche in den stenotischen Bereich eingeschoben werden und dort durch ihre Eigensteifigkeit das Gefäßlumen offenhalten. Auch ist es bekannt, solche Stents zum Verschluß von Gefäßwandaussackungen (Aneurysmen) oder Fisteln einzusetzen.

Herkömmlicherweise wurden dabei ballondilatierbare Stents eingesetzt. Diese werden zur Einbringung in nicht-dilatiertem Zustand auf einen nicht expandierten Ballon aufgekrimpt, über ein Kathetersystem an die zu behandelnde Stelle geführt und dort durch Expansion des Ballons dilatiert und so im Gefäß verankert. Da zur Einführung ballondilatierbarer Stents keine aufwendigen Stütz- und Überhüllen notwendig sind, können diese auch in sehr feine Gefäße eingeführt werden. Problematisch ist jedoch, daß sie aufgrund ihrer plastischen Verformbarkeit durch Druckeinwirkung von außen einfach zusammengedrückt werden können. Ein weiterer Nachteil besteht darin, daß sie zur Verankerung mittels Beaufschlagung mit hohem Druck zunächst über den Umfang ausgedehnt werden müssen, den sie letztlich einnehmen. Diese Aufweitung über den notwendigen Umfang birgt die Gefahr einer Gefäßverletzung, was die Bildung von Thromben nach sich ziehen kann.

Zur Vermeidung dieser Risiken ist es bekannt, selbstexpandierende Stents einzusetzen, die aus Formgedächtnismaterialien gefertigt sind. Diese besitzen eine geflechtartige Struktur und werden zunächst in kollabiertem Zustand durch einen Katheter an den Zielort geführt, wo sie sich infolge der Temperaturveränderung (thermischer Formgedächtniseffekt) oder durch den Wegfall des durch den Katheter ausgeübten mechanischen Zwanges (Superelastizität) aufweiten. Solche Stents weisen den Nachteil auf, daß die zur Einführung notwendigen Vorrichtungen relativ aufwendig und platzintensiv sind. (So ist bei den bekannten superelastisch expandierbaren Stents stets eine Stütz- und Überhülle nötig, was einen relativ großen Katheterumfang bedingt.)

Für den Einsatz in besonders kleinlumigen intrakraniellen Gefäßen ist es daher bekannt, Stents aus Formgedächtnismaterial einzusetzen, die zunächst als langgestrecktes Filament vorliegen und erst bei Austritt aus dem Katheter durch die Temperaturveränderung oder den Wegfall des zuvor durch den Katheter ausgeübten Zwanges die röhrenförmige Struktur eines Stents annehmen.

So ist es beispielsweise aus der DE 197 03 482 bekannt, zur Behandlung von Aneurysmen und dergleichen einen Stent aus zwei langgestreckten Filamenten einzusetzen, welche durch den mechanischen Zwang des Katheters spannungsinduziert in der langgestreckten Form gehalten werden und nach Wegfall dieses Zwanges, bei Herausschieben aus dem Katheter die eigentliche Stent-Form einnehmen. Hierdurch wurde erstmals der Einsatz von Stents mit Formgedächtniseigenschaften auch in sehr kleinlumigen Gefäßen, wie den intrakraniellen und cerebralen Gefäßästen möglich.

Diese Stents weisen jedoch den Nachteil auf, daß sie im Katheter relativ schwer verschiebbar sind, da sie notwendigerweise von innen Druck auf die Katheterinnenwand ausüben. Zudem ist es notwendig, sie bereits ihre Zielposition bereits vor Ausschieben aus dem Stent exakt zu bestimmen, da sie sofort beim Eintritt in das Blutgefäß die Stent-Form annehmen. Diese ist im Gefäß nur schwer verschieblich. Zudem kann der aufgeweitete Stent nach Einnahme der bestimmungsgemäßen Stent-Form nicht mehr in den Katheter zurückgeführt werden kann, dessen Innenlumen notwendigerweise kleiner dimensioniert ist als der Außenumfang des Stents, da er zuvor die wesentlich dünner dimensionierte, filamentöse Grundform des Stents in dieser Form halten mußte.

Die im wesentlichen gleiche Problematik findet sich bei anderen endovaskulären Implantaten aus diesen Materialien.

Die WO 92/22263 A2, die den Oberbegriff des Anspruchs 1 bildet, beschreibt ein Nitinolstentsystem, bei dem der Stent aus einem zur Wendel geformten Stahlband mit Hilfe eines Dorns/Drahtes in einer engen Konfiguration gehalten wird, aus der er nach Entfernen des Dorns/Drahtes in eine weitere Konfiguration aufweitet, entsprechend einer aufgezogenen und entspannten Spiralfeder. Der Stent unterscheidet sich folglich in seiner primären und sekundären Struktur nur durch seinen Durchmesser.

Angesichts der mit dem Stand der Technik verbundenen Nachteile besteht die Aufgabe der Erfindung in der Bereitstellung von Implantaten, welche auch in kleinlumige Gefäße eingebracht werden können und zudem gut positionierbar sind.

Diese Aufgabe wird erfindungsgemäß durch das medizinische Implantat gemäß Anspruch 1 gelöst.

Die zur übergeordneten Struktur vorgeformte primäre Wendel wird dabei durch das, beispielsweise als im wesentlichen gestreckter Draht ausgebildete, Halteelement unter elastischer Vorspannung oder in spannungsinduziertem martensitischen Zustand gehalten. Nach Entfernung des Halteelementes von dem Filament fällt dieser Zwang weg, so daß die primäre Wendel ihre vorbestimmte, übergeordnete Struktur einnehmen kann. Da der Zwang hier nicht von außen (also z. B. durch den umgebenden Katheter) ausgeübt wird, sondern von innerhalb der primären Wendel, ist das erfindungsgemäße Implantat innerhalb des Katheters wesentlich besser manövrierbar und kann auch außerhalb des Katheters noch repositioniert werden, bis es den idealen Bestimmungsort im Organismus erreicht hat.

Die Begriffe "proximal" und "distal" werden im folgenden so verstanden, daß sich "proximal" auf die Ausrichtung nach außerhalb des Zielorganismus, also zum

Operateur hin bezieht, "distal" weist dagegen von dem Operateur weg auf den Zielort innerhalb des Organismus hin.

Zur Implantation wird das Implantat dabei vorzugsweise auf eine Einführhilfe aufgesetzt und mittels eines Mikrokatheters an den Implantationsort manövriert. Einführhilfe und Halteelement sind so dimensioniert, daß sie vom Operateur separat bedienbar sind. In der einfachsten Form sind sie einfach als Längenelemente ausgebildet, die nach proximal durch den Mikrokatheter bis zum Operateur reichen und dort von ihm zusammen mit dem Mikrokatheter manipuliert werden können. Nach Erreichen des Zielortes wird das erfindungsgemäße Implantat mit dem darin befindlichen Halteelement nach distal aus dem Mikrokatheter ausgeschoben und unter beispielsweise röntgenologischer Kontrolle exakt positioniert. Nach Einnahme der bestimmungsgemäßen Position wird das Halteelement entfernt, so daß das Implantat die durch die übergeordnete Struktur bedingte Form einer sekundären Wendel annimmt. Auch nach Entfernung des Halteelementes und Einnahme der bestimmungsgemäßen Form kann es mit Hilfe der Einführhilfe noch repositioniert und bei entsprechender Dimensionierung des Mikrokatheter-Innenlumens sogar wieder in diesen zurückgeführt werden.

Grundsätzlich ist die durch die übergeordnete Struktur bestimmte, bestimmungsgemäße Form des Implantates, abhängig vom Einsatzzweck, frei wählbar. So sind beispielsweise korb- oder röhrenförmige Strukturen für den Einsatz beim Verschluß von Gefäßanomalien besonders zweckmäßig. Gemäß einer bevorzugten Ausführungsform führt die Ausbildung der übergeordneten Struktur zur Ausbildung einer im wesentlichen röhrenförmigen Form. Das lmplantat ist dann insbesondere als Stent einsetzbar. Generell stellt die übergeordnete Form eine Wendel, bevorzugt eine Spiralwendel dar. Diese Strukturen können die für Stents typische, im wesentlichen röhrenförmige Gestalt aufweisen und sind zudem besonders flexibel und stabil.

Die Dimensionierung der Implantate hängt vom Zielgefäß ab und kann vom zuständigen Fachmann einfach bestimmt werden. Für den Einsatz in feinen intrakraniellen oder cerebralen Gefäßen eignen sich besonders die implantate mit Wendelstruktur, die einen Durchmesser von 0,5 bis 10 mm aufweisen.

Es ist besonders zweckmäßig, wenn das Filament des erfindungsgemäßen Implantates eine in Längsachse verlaufende Ausnehmung zur Aufnahme des Halteelementes aufweist. Dabei liegt das Filament in seiner Grundform vorzugsweise selbst als Wendel vor, deren Lumen zur Aufnahme des Halteelementes dient und zweckmäßigerweise zum distalen Ende hin geschlossen ist, so daß das Halteelement dort nicht heraustreten kann. Das Filament selbst stellt eine primäre Wendel dar. Die von dem Filament nach Wegfall des durch das Halteelement ausgeübten mechanischen Zwanges eingenommene übergeordnete Struktur stellt dann die sekundäre Struktur dar (also die sekundäre Wendel). Das Halteelement ist vorzugsweise lose, zumindest jedoch einfach lösbar, in der Ausnehmung des Filamentes angeordnet, so daß sie einfach vom Filament entfernt werden kann. Besonders zweckmäßig, da besonders atraumatisch ist eine Ausführungsform, bei der das Filament, insbesondere in seiner Ausführung als primäre Wendel/Feder am distalen Ende abgerundet ist.

Zur Ausbildung der primären Wendel eignen sich insbesondere metallische Drähte mit einem Durchmesser von 0,03 bis 0,3 mm und vorzugsweise von 0,05 und 0,2 mm.

Gemäß einer besonders bevorzugten Ausführungsform ist das Filament zumindest teilweise aus einem Material mit Formgedächtniseigenschaften ausgebildet. Zweckmäßig ist hier als Material eine metallische Legierung mit der Fähigkeit, die spannungsinduzierte martensitische Transformation zu durchlaufen. Besonders bevorzugt sind dabei solche Legierungen, die gleichzeitig die Fähigkeit aufweisen, eine temperaturinduzierte martensitische Transformation zu durchlaufen. Hier eignen sich insbesondere Titan und Nickel enthaltende Legierungen sowie Eisenbasis- oder Kupferbasislegierungen.

Der Begriff "Formgedächtnis" ist dem angesprochenen Durchschnittsfachmann bekannt. Er umfaßt sowohl das mechanisch als auch das thermisch induzierte Formgedächtnis. Als Materialien mit Formgedächtniseigenschaften werden im Rahmen dieser Erfindung sowohl Materialien verstanden, die entweder ein thermisches oder ein mechanisches Formgedächtnis aufweisen, wie auch solche Materialien mit thermisch und mechanisch induziertem Formgedächtnis.

Diese Materialien weisen dabei die Fähigkeit auf, abhängig von der Temperatur zwischen einem eher rigiden und einem sehr flexiblen Zustand hin und her zu wechseln, wobei sie auch Übergangszustände durchlaufen. Sie sind wesentlich stärker durch Biegung und Zug beanspruchbar als herkömmliche Werkstoffe. Das Material kann dabei insbesondere im flexiblen Zustand extrem stark verbogen und gedehnt werden, ohne zu reißen. Erst bei Erhöhung der Temperatur kehrt es in seinen rigiden Zustand zurück, was im Falle einer vorangegangenen Verformung mit einer Formänderung einhergeht. Die jeweilige Temperaturschwelle kann, in für den Zuständigen Fachmann bekannter Weise, anhand der Zusammensetzung des Materials gesteuert werden.

Es ist zweckmäßig, wenn das erfindungsgemäße Implantat auch eine metallische Legierung mit Formgedächtniseigenschaften enthält und vorzugsweise im wesentlichen daraus besteht. Dies können Legierungen sein, welche nur die Fähigkeit zur spannungsinduzierten martensitischen Transformation haben, vorzugsweise handelt es sich dabei jedoch um solche Legierungen, welche sowohl die Fähigkeit zur spannungsinduzierten als auch zur temperaturinduzierten martensitischen Transformation aufweisen. Hier eignen sich insbesondere Titan und Nickel enthaltende Legierung en sowie Eisenbasis- und Kupferbasislegierungen.

Titan-Nickel Legierungen weisen dabei, abhängig von der Temperatur unterschiedliche Kristallstrukturen auf: Die bei hoher Temperatur vorliegende Phase wird als Austenit bezeichnet. Ihre Atomanordnung ist kubisch flächenzentriert; sie stellt die stabile Phase dar. Bei niedriger Temperatur liegen die Atome in einer solchen Legierung in tetragonal verzerrter, kubisch raumzentrierter Anordnung vor. Sie wird als Martensit bezeichnet. Die durch die Temperatur bedingte Martensitphase wird auch als temperaturinduzierter Martensit (TIM) bezeichnet. Durch die Wahl der Legierungszusammensetzung kann dabei bestimmt werden, bei welcher Temperatur ein Übergang (Transformation) von der einen zur anderen Phase erfolgt, dies kann über einen Bereich von -100 bis 100 °C erfolgen.

Wirkt während der Umwandlung von Austenit in Martensit (infolge einer Senkung der Temperatur unter einen kritischen Wert) keine äußere Kraft ein, ist keine makroskopische Gestaltsänderung zu beobachten. Im martensitischen Zustand ist das Bauteil leicht zu verformen, wobei eine Gestaltsänderung von bis zu ca. 8 % erreicht werden kann. Solange das Material unter der kritischen Temperaturschwelle (der Umwandlungstemperatur) bleibt, ist die Verformung stabil. Wird der verformte Martensit jedoch erwärmt, stellt sich bei Überschreiten der Umwandlungstemperatur die ursprüngliche Gestalt wieder ein. Dieses, durch unterschiedliche Umgebungstemperaturen gesteuerte Formgedächtnis des temperaturinduzierten Martensits wird auch als thermisches Formgedächtnis (Shape Memory) bezeichnet.

Neben diesem thermischen Formgedächtnis können metallische Legierungen auch ein mechanisches Formgedächtnis (Superelastizität) aufweisen, welches auf der Einnahme einer spannungsinduzierten martensitischen Phase (SIM) beruht: In bestimmten Temperaturbereichen, welche für den Fachmann durch Wahl der Legierungszusammensetzung einstellbar sind, kann der Übergang in die martensitische Phase auch mechanisch durch Einwirkung eines äußeren Zwanges induziert werden (spannungsinduzierter Martensit). Auf diese Weise können Dehnungen bis zu 10 % erreicht werden. Bleibt das Material bei dieser Temperatur, welche über der Temperaturschwelle der Umwandlung von Martensit zu Austenit liegt, so geht es wieder in die austenitischen Phase über, es kommt zur Rückverformung.

Die thermische Umwandlung von Martensit zu Austenit findet jedoch innerhalb eines Temperaturbereiches, nicht bei Überschreiten eines streng begrenzten Temperaturwertes statt, so daß es Übergangsphasen in der Materialstruktur gibt. Fällt nun die Einwirkung einer mechanischen Spannung auf einen spannungsinduzierten Martensit bei einer Temperatur in diesem Zwischenbereich weg, so kommt es zu einer teilweisen, spannungsbedingten Rückwandlung zu Austenit und somit zu einer teilweisen Rückverformung. Erst bei einem Anstieg der Temperatur kommt es zur vollständigen Umwandlung in die Austenitphase. In diesem Falle liegt eine Kombination aus spannungsinduzierter und temperaturinduzierter Phasenumwandlung vor.

Aufgrund der besonders guten technologischen Eigenschaften, die solche metallischen Legierungen im martensitischen Zustand (d. h. im temperatur- und/oder spannungsinduzierten martensitischen Zustand) aufweisen, wird das zur Fertigung des erfindungsgemäßen Implantates verwendete Material vorzugsweise so gewählt, daß das Implantat durch das Haltemittel in spannungsinduziertem Martensit gehalten wird. Besonders bevorzugt ist die Wahl von Legierungen mit beiden Effekten, so daß das Filament durch den Anstieg der Umgebungstemperatur bei Austritt aus dem Katheter und Einführung in das Blutgefäß und den Wegfall der Spannung nach Entfernung des Halteelementes eine gemischt austenitische-martensitische Transformation durchläuft.

Zur Nutzung des mechanisch induzierten Formgedächtnisses im Körper eignen sich insbesondere Legierungen mit einer Umwandlungstemperatur von -15 °C bis +38 °C und insbesondere von -15 °C bis + 20 °C. Zur Nutzung des thermisch induzierten Formgedächtnisses im Körper eignen sich insbesondere Legierungen mit einer Umwandlungstemperatur von + 35 bis +38 °C. Die zur Induzierung von Formgedächtniseffekten, insbesondere auch gemischter (SE und TIM) Effekte im Körper besonders geeigneten Umwandlungstemperaturen sind dem zuständigen Fachmann hinlänglich bekannt.

Zur Ausbildung des Halteelementes ist grundsätzlich jedes Material genügend hoher Stabilität und Zugfestigkeit geeignet. Die Wahl des Materials und die Dimensionierung des Durchmessers des Halteelementes beeinflussen einander und sind ebenso abhängig von den Materialeigenschaften und dem Durchmesser des Filamentes. Diese Zusammenhänge sind dem zuständigen Durchschnittsfachmann geläufig, so daß er jeweils geeigneten Durchmesser und das geeignete Material bestimmen kann. In Versuchen der Erfinder haben sich medizinische Edelstahldrähte verschiedener Durchmesser bewährt.

Besonders zweckmäßig ist ein Implantat, dessen übergeordnete Struktur eine Wendel bildet, bei der die Steigungen der Wendelschlaufen über die Länge des Implantates variieren.

So ist es zur Abdichtung von Aneurysmen besonders zweckmäßig, wenn die Steigungen von der Mitte des Implantates zu den Enden hin abnehmen, so daß sich in der Mitte eine dichtere und an den Enden eine losere Anordnung der Wendelschlaufen ergibt. Das Implantat wird dann mit dem dichten mittleren Bereich vor den Aneurysmeneingang plaziert. Diese Ausführungsform ist überdies deshalb besonders zweckmäßig, weil der mittlere Bereich des Implantates eine relativ hohe Röntgendichte hat und als radiopaquer Marker dienen kann.

Gemäß einer bevorzugten Ausführungsform ist es möglich, das Implantat/den Stent in an sich bekannter Weise mit medizinisch wirksamen Substanzen, etwa thrombosierungshemmenden Wirkstoffen, beschichtet sein.

Die Erfindung betrifft weiterhin eine Vorrichtung zur Einbringung von Implantaten in Körpergefäßen und Hohlräumen mit einem Implantat gemäß vorstehender Erläuterung und einer Einführhilfe, welche lösbar mit dem proximalen Ende des Implantates verbunden ist.

Es ist dabei besonders vorteilhaft, wenn die Einführhilfe als Röhre (oder ein anderes Längenelement mit in Längsachse verlaufender Ausnehmung) ausgebildet ist, durch deren Lumen sich das Halteelement von dem Implantat nach proximal hin erstreckt. Das Halteelement ist dabei zweckmäßigerweise als Streckdraht, insbesondere aus medizinischem Edelstahl ausgebildet.

Einführhilfe und Implantat können dabei direkt oder über ein Ablösemodul miteinander verbunden sein. Geeignet ist jede im Körper lösbare Verbindung. Die Verwendung eines separaten Ablösemodules ermöglicht die Verwendung einfach konfektionierbarer Einheitsmodule als Einführhilfe, Implantat und Ablösemodul und ist daher besonders kostengünstig.

Die erfindungsgemäße Vorrichtung eignet sich grundsätzlich für jede Art der Ablösung des Implantates, beispielsweise die mechanische, thermische oder elektrochemische. Diese Ablösemechanismen und die zugehörige Technik sind dem zuständigen Fachmann geläufig. Ebenso die dazu nötige Ausgestaltung der lösbaren Verbindung zwischen Einführhilfe und Implantat bzw. des Ablöseelementes oder sonstige notwendige Einrichtungen der erfindungsgemäßen Vorrichtung.

Gemäß einer bevorzugten Ausführungsform ist die Vorrichtung für die elektrochemische Ablösung des Implantates ausgerüstet. Dazu weist sie weiterhin einen Katheter, eine Spannungsquelle und eine Kathode auf. Der Katheter ist dabei elektrisch isolierend ausgebildet oder die Einführhilfe ist selbst zumindest in ihrem distalen Bereich (z. B. mittels einer geeigneten Beschichtung oder durch Überzug mit einem Schrumpfschlauch) isoliert. Das Implantat dient dabei als Anode und ist im Katheter in Längsrichtung verschiebbar. Die Verbindung von Implantat und Einführhilfe weist eine elektrolytisch korrodierbare Stelle auf, so daß das Implantat im Kontakt mit einer Körperflüssigkeit durch elektrolytische Prozesse abtrennbar ist. Alternativ oder zusätzlich dazu können eine oder mehrere Ablösestellen im Implantat selbst angeordnet sein, beispielsweise gleichmäßig über dessen Länge verteilt, so daß sich, in dem Maße, wie das Implantat aus dem Katheter hinaustritt, ein oder mehrere Segmente an der jeweils katheternächsten Ablösestelle abtrennen lassen. Auf diese Weise ist es möglich, variable Längen des Implantats abzutrennen, wobei ein oder mehrere intakte Ablösestellen im Implantat verbleiben können, oder mehrere Implantate im Zuge einer Behandlung nacheinander zu plazieren. Dies kann insbesondere bei der Behandlung von Gefäßmißbildungen an Bifurkationen vorteilhaft sein.

Ist die Verbindung zwischen Einführhilfe und Implantat als separates Ablösemodul ausgebildet, so ist es besonders zweckmäßig, wenn das Ablösemodul mit dem Implantat bzw. der Einführhilfe durch Verschweißen, Verlöten, Verkleben oder mechanische Fügeverfahren verbunden ist. Besonders vorteilhaft ist dabei ein Ablösemodul, welches eine proximale und eine distale Wendel sowie ein dazwischenliegendes Segment aufweist, das die elektrolytisch korrodierbare Stelle ausbildet.

Die Wendeln des Ablösemoduls sind dabei einerseits mit der Einführhilfe und andererseits mit dem Implantat fest verbunden, vorzugsweise durch Verschweißen, Verkleben oder mechanische Fügeverfahren. Nach der Korrosion der elektrolytisch korrodierbaren Stelle wird so die distale Wendel mit dem Implantat abgelöst und implantiert. Bei dieser Ausführungsform der erfindungsgemäßen Vorrichtung ist es daher zweckmäßig, wenn die Wendeln des Ablösemoduls aus einem besonders biokompatiblen und atraumatischen Material, insbesondere einer Platinlegierung bestehen. Die Dimensionierung des Ablösemoduls bzw. der mit implantierten Wendel wird dabei so gewählt, daß sie nur einen geringen Bruchteil der Länge des Implantates ausmacht und so bei der Implantation nicht stört.

Die elektrolytische Korrosion der Stelle wird dabei durch Wahl geeigneter Materialkombinationen von Wendeln und zwischenliegendem Segment gewährleistet. Diese sind dem Fachmann hinlänglich bekannt. Diesbezüglich wird auf die Schrift WO 01/32085 A1 verwiesen.

Vorzugsweise enthalten die elektrolytisch nicht-korrodierbaren Abschnitte bzw. Wendeln des Ablösemodules eines oder mehrere der folgenden Materialien: Edelmetalle oder Edelmetall-Legierungen, korrosionsbeständige Keramikwerkstoffe, korrosionsbeständige Kunststoffe, vorzugsweise Platinmetall-Legierungen (insbesondere Pt/Ir).

Ebenso bevorzugt ist eine Ausbildung der erfindungsgemäßen Vorrichtung, deren lösbare Verbindung bzw. Ablösemodul an der elektrolytisch korrodierbaren Stelle eines oder mehrere der folgenden Materialien enthält: Keramikwerkstoffe, Kunststoffe, Nichtedelmetalle oder Legierungen derselben, vorzugsweise rostfreier Stahl. Hierbei sind insbesondere die nicht rostenden Stähle der Typen AISI 301, 303 oder 316 bzw. Untergruppen dieser Typen geeignet. Die für die Ausbildung der Verbindung bzw. des Ablösemodules verwendeten Keramikwerkstoffe und Kunststoffe sind elektrisch leitfähig.

In einer vorteilhaften Ausführungsform werden für die Ausbildung der elektrolytisch nicht-korrodierbaren Wendeln der Ablösemodule an den Übergängen zu den elektrolytisch korrodierbaren Stellen Materialkombinationen gewählt, die dazu geeignet sind, Lokalelemente auszubilden. Auf diese Weise wird - unabhängig von der Verringerung des Durchmessers der korrodierbaren Stellen - die elektrolytische Ablösbarkeit des Occlusionsmittels verbessert.

Hierbei eignen sich am besten Materialkombinationen, in denen zur Ausbildung der elektrolytisch korrodierbaren Stellen nicht rostende Stähle, vorzugsweise der Typen AISI 301, 304, 316 oder Untergruppen derselben, Ti oder TiNi-Legierungen oder Co-Basislegierungen mit einem oder mehreren der folgenden Edelmetalle bzw. Edelmetall-Legierungen: Pt, Pt-Metalle, Pt-Legierungen, Au-Legierungen oder Sn-Legierungen.

In einer weiteren vorteilhaften Ausführungsform ist die Spitze der Einführhilfe beispielsweise durch eine schlecht korrodierbare Materialbeschichtung oder Überzug mit einem Schrumpfschlauch isoliert, so daß sie nicht elektrolytisch korrodierbar ist.

Die erfindungsgemäße Vorrichtung ist vorzugsweise für den Einsatz in tier- oder humanmedizinischen Verfahren, besonders bei der endovaskulären Behandlung intrakranieller Aneurysmen und erworbener oder angeborener arteriovenöser Gefäßmißbildungen und/oder -fisteln und/oder Tumorembolisation durch Thrombosierung bestimmt. Das Implantat ist dabei in seiner bestimmungsgemäßen Form vorzugsweise als Stent ausgebildet, kann aber auch jede andere zweckmäßige übergeordnete Struktur einnehmen.

Die Erfindung wird nachfolgend beispielhaft anhand der in den Zeichnungen veranschaulichten Ausführungsbeispiele näher erläutert. Es stellen dar:
- Figur 1: die Seitenansicht eines erfindungsgemäßen Implantates in gestreckter, filamentöser Form und vergrößerter Darstellung;
- Figur 2: ebenfalls in vergrößerter Darstellung und Seitenansicht die erfindungsgemäße Vorrichtung mit gestrecktem Implantat.

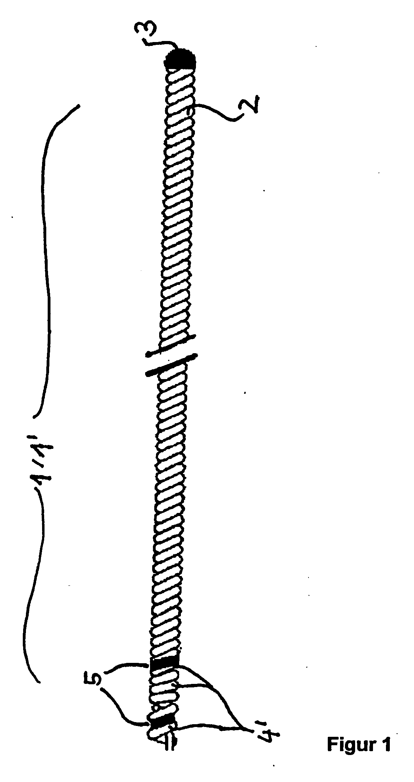

In Figur 1 ist in vergrößerter Seitenansicht ein Ausschnitt eines erfindungsgemäßen Implantates 1 gezeigt. Das distale Ende befindet sich auf der rechten, das proximale auf der linken Seite der Figurendarstellung. In dem Implantat 1 ist (nicht sichtbar) lose ein als Streckdraht aus medizinischem Edelstahl ausgebildetes Halteelement angeordnet, welches das Implantat 1 in seiner gestreckten Form als primäre Wendel 1' hält. Das Implantat 1 ist aus einem Draht 2 einer Titan-Nickel Legierung ausgebildet, welche sowohl mechanische als auch thermische Formgedächtniseigenschaften aufweist.

Der Draht 2 weist einen Durchmesser von 0,06 mm auf und ist zu einer primären Spiralwendel mit 0,2 mm Durchmesser aufgewickelt. Die Spiralwendel endet nach distal in einer abgerundeten Spitze 3 aus einer Platin/Iridium Legierung. Diese Ausbildung ist besonders atraumatisch. Gleichzeitig dient die Spitze 3 dabei als distaler Implantatmarker zur röntgenologischen Kontrolle der Positionierung. Am proximalen Ende ist das Filament 1' an zwei Nähten 5 fest mit der distalen Wendel 4 eines Ablösemoduls verschweißt. Die distale Wendel 4 ist ihrerseits fest mit einem dünnen Draht 6 aus Edelstahl verschweißt, welcher die elektrolytisch korrodierbare Stelle ausbildet.

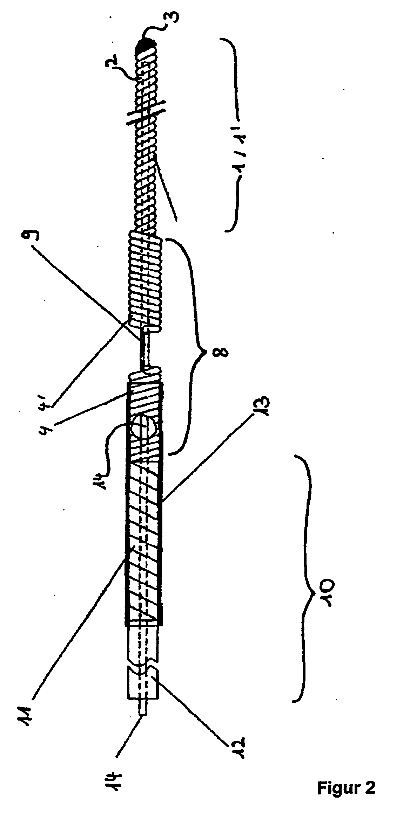

Die Figur 2 zeigt eine andere beispielhafte Ausführungsform des erfindungsgemäßen Implantates 1, welches in dieser Figur als Teil einer erfindungsgemäßen Implantationsvorrichtung 7 dargestellt ist. Das Filament 1' ist auch hier als primäre Spiralwendel aus einem Titan-Nickel-Draht 2 ausgebildet und weist eine abgerundete distale Spitze 3 aus einer Pt/lr-Legierung auf. Das sich nach proximal an das Filament 1' anschließende Ablösemodul 8 umfaßt eine proximale und eine distale Wendel 4 und 4' aus Pt/Ir-Draht sowie ein dazwischenliegendes, an die Wendeln 4/4' geschweißtes Segment 9 aus medizinischem Edelstahl, welches die elektrolytisch korrodierbare Stelle ausbildet. Nach proximal ist das Ablösemodul 8 durch schweißtechnische Verbindung seiner proximalen Wendel 4 mit dem distalen Ende der Einführhilfe 10 verbunden. Die Einführhilfe ist im distalen Bereich als Drahtspirale 11 ausgebildet (hier eine Flachbandspirale) und in ihrem proximalen Bereich als Kanülenrohr 12. Der distale Bereich der Einführhilfe 10 sowie ein Teil des Ablösemoduls 8 sind durch einen Schrumpfschlauch 13 nach außen elektrisch isoliert.

Das als Streckdraht ausgebildete Halteelement 14 läuft vom proximalen Ende der Vorrichtung bis in den distalen Bereich der primären Spirale des Filamentes 1' und durchspannt dabei die Einführhilfe 10, das Ablösemodul 8 sowie die Länge des Filamentes 1', wie anhand seiner teilweise gestrichelten Darstellung sichtbar ist (die runde Ausnehmung der proximalen Wendel 4 ist nicht Bestandteil der Vorrichtung und dient lediglich der besseren Darstellung). Die Vorrichtung 7 wird in einem Mikrokatheter an den Implantationsort manövriert. Dort wird das Implantat 1 als gestrecktes Filament durch Verschieben der Einführhilfe 10 in das Blutgefäßsystem vor den Aneurysmeneingang plaziert. Der Streckdraht 14 wird ebenfalls mitgeführt.

Die korrekte Positionierung wird dabei durch röntgenologische Kontrolle des Spitzenmarkers 3 sowie, der ebenfalls röntgendichten Wendeln 3/3' des Ablösemoduls 8 überwacht. Nach Einnahme der gewünschten Position wird dieser durch einfaches Herausziehen aus dem Filament 1' entfernt, infolgedessen es durch den Wegfall der durch diesen ausgeübten Spannung zur Ausbildung der übergeordneten Struktur, also einer sekundären Spiralwendel kommt (nicht dargestellt), welche als Stent dient.

Die Spiralwendel ist in diesem Beispiel so ausgebildet, daß die Dichte ihrer Wicklungen von der Mitte zu ihren Enden abnimmt. Diese Ausführungsform eignet sich besonders gut zum Verschluß von Aneurysmen. Überdies kann anhand des von dem röntgendichteren mittleren Bereich geworfenen Röntgenschatten bei der Operation die korrekte Positionierung des Implantates auch nach der Ausbildung der übergeordneten Struktur überprüft werden. Weicht die tatsächliche Positionierung von der optimalen ab, so können mit Hilfe der Einführhilfe 10 noch Positionskorrekturen des Implantates vorgenommen werden. Das Implantat ist auch nach Ausbildung der übergeordneten Struktur elastisch genug, um in mehr oder weniger gestreckter Form wieder in den Katheter zurückgezogen zu werden, so daß beispielsweise bei einer Fehlplazierung oder Fehldimensionierung der übergeordneten Struktur eine komplette Rückführung des Implantates 1 aus dem Blutgefäßsystem möglich ist.

Ist jedoch die optimale Positionierung des fertig ausgebildeten Stents gegeben, wird dieser durch Anlegen einer Spannung mit Hilfe einer Spannungsquelle durch elektrolytische Korrosion der elektrolytisch korrodierbaren Stelle 9 von der Einführhilfe 10 abgetrennt. Das als Stent dienende Implantat 1 verbleibt dann im Blutgefäß, wo es den Verschluß des Aneurysmas herbeiführt, wohingegen der Rest der Vorrichtung 7 aus dem Organismus entfernt wird.

## Patentansprüche

1. Medizinisches Implantat in Form mindestens einer langgestreckten primären Wendel (1'), wobei die primäre wendel (1') zur Einnahme einer übergeordneten Struktur vorgeformt ist, die sie am Implantationsort während der Implantation einnimmt, die primäre Wendel (1') in Längsachse von mindestens einem Halteelement (14) durchzogen ist, das aus dieser entfernbar ist, **dadurch gekennzeichnet** wobei daß die primäre wendel (1') bis zu dessen Entfernung an der Einnahme der übergeordneten Struktur gehindert wird, und daß die übergeordnete Struktur eine sekundäre wendel ist.

2. Medizinisches lmplantat (1) gemäß Anspruch 1, **dadurch gekennzeichnet, daß** die übergeordnete Struktur eine im wesentlichen tubuläre Form ausbildet.

3. Medizinisches Implantat (1) gemäß Anspruch 2, **dadurch gekennzeichnet, daß** die sekundäre Wendel einen Außendurchmesser von 0,5 bis 10 mm aufweist.

4. Medizinisches Implantat (1) gemäß Anspruch 1, **dadurch gekennzeichnet daß** die primäre Wendel (1') einen Außendurchmesser von 0,1 bis 0,5 mm aufweist.

5. Medizinisches Implantat (1) gemäß Anspruch 1, **dadurch gekennzeichnet, daß** die primäre Wendel (1') aus einem Draht (2) mit einem Durchmesser von 0,03 bis 0,3 mm und vorzugsweise zwischen 0.05 und 0,2 mm besteht.

6. Medizinisches Implantat (1) gemäß einem, der vorstehenden Ansprüche, **dadurch gekennzeichnet, daß** die Wendel (1) zumindest teilweise aus einem Material mit Formgedächtniseigenschaften besteht.

7. Medizinisches Implantat (1) gemäß Anspruch 6, **dadurch gekennzeichnet, daß** das Material eine metallische Legierung mit der Fähigkeit ist, eine spannungsinduzierte martensitische Transformation zu durchlaufen.

8. Medizinisches Implantat (1) gemäß Anspruch 7, **dadurch gekennzeichnet, daß** die Legierung die Fähigkeit aufweist, eine temperaturinduzierte martensitische Transformation zu durchlaufen.

9. Medizinisches Implantat (1) gemäß einem der Ansprüche 6 oder 7, **dadurch gekennzeichnet, daß** die Legierung eine Titan und Nickel enthaltende Legierung, eine Eisenbasis- oder Kupferbasislegierurtg ist.

10. Medizinisches Implantat (1) gemäß einem der vorstehenden Ansprüche, **dadurch gekennzeichnet, daß** das Halteelement (14) ein metallischer Draht, vorzugsweise ein Draht aus medizinischem Edelstahl ist.

11. Medizinisches Implantat (1) gemäß einem der Anspruche 1 bis 10, **dadurch gekennzeichnet, daß** die Steigungen der durch die übergeordnete Struktur gebildeten Wendelschlaufen über die Länge des Implantates (1) variieren.

12. Medizinisches Implantat (1) gemäß Anspruch 11, **dadurch gekennzeichnet, daß** die Steigungen von der Mitte zu den Enden hin abnehmen.

13. Medizinisches Implantat nach einem der vorstehenden Ansprüche, **gekennzeichnet, durch** wenigstens ein darin angeordnetes Ablösemodul mit einer elektrolytisch korrodierbaren Stelle.

14. Medizinisches Implantat nach Anspruch 13, **dadurch gekennzeichnet, daß** es in regelmäßigen Abständen angeordnete Ablösemodule aufweist.

15. Vorrichtung (7) zur Implantation von Implantaten in Körpergefäßen und Hohlräumen mit einem Implantat (1) gemäß einem der vorstehenden Ansprüche 1 bis 14 und einer Einführhilfe (10), welche lösbar mit dem proximalen Ende des Implantates (1) verbunden ist.

16. Vorrichtung (7) gemäß Anspruch 15, **dadurch gekennzeichnet, daß** die Einführhilfe (10) als Röhre ausgebildet ist, durch deren Lumen sich das Halteelement (14) von dem Implantat (1) nach proximal hin erstreckt.

17. Vorrichtung (7) gemäß Anspruch 16, **dadurch gekennzeichnet, daß** Implantat (1) und Einführhilfe (10) durch ein Ablösemodul (8) miteinander verbunden sind.

18. Vorrichtung (7) gemäß einem der Ansprüche 15 bis 17 mit einem Katheter, einer Spannungsquelle und einer Kathode, bei der das lmplantat (1) als Anode dient und im Katheter in Längsrichtung verschiebbar ist, wobei die Verbindung von Implantat (1) und Einführhilfe (10) eine elektrolytisch korrodierbare Stelle (9) aufweist, so daß das Implantat (1) im Kontakt mit einer Körperflüssigkeit durch elektrolytische Prozesse abtrennbar ist.

19. Vorrichtung (7) gemäß Anspruch 18, **dadurch gekennzeichnet, daß** das Ablösemodul (8) eine proximale und eine distale Wendel (4/4') sowie ein dazwischenliegendes Segment (9) umfaßt, wobei die Wendeln (4/4') aus Material bestehen, welches gegenüber der elektrolytischen Korrosion weniger anfällig ist als das des zwischenfiegenden Segmentes (9).

20. Vorrichtung (7) gemäß Anspruch 19, **dadurch gekennzeichnet, daß** das Ablösemodul (8) mit dem Implantat (1) bzw. der Einführhilfe (10) durch Verschweißen, Verlöten, Verkleben oder mechanisches, insbesondere kraft- bzw. formschlüssiges Fügen unlösbar verbunden ist

21. Vorrichtung (7) gemäß einem der Ansprüche 15 bis 20, **dadurch gekennzeichnet, daß** die Einführhilfe (10) zumindest teilweise als Wendel oder Feder ausgebildet ist.

22. Vorrichtung (7) gemäß einem der Ansprüche 15 bis 20, **dadurch gekennzeichnet, daß** die Einführhilfe (10) zumindest teilweise von einem elektrisch isolierenden Schrumpfschlauch oder einer elektrisch isolierenden Beschichtung umgeben ist.

## Claims

1. Medical implant in form of at least one elongated primary coil (1'), said primary coil (1') being preformed so as to assume a superimposed structure, which it assumes during implantation at the placement site, and said primary coil (1') comprising at least one retaining element (14) passing through its longitudinal axis and being removable from said primary coil (1'), **characterized in that** said retaining element prevents the primary coil (1') from assuming said superimposed structure and that the superimposed structure is a secondary coil.

2. The medical implant (1) according to claim 1, **characterized in that** the superimposed structure has a primarily tubular form.

3. The medical implant (1) according to claim 2, **characterized in that** the secondary coil has an outer diameter ranging between 0.5 and 10 mm.

4. The medical implant (1) according to claim 1, **characterized in that** the primary coil (1') has an outer diameter ranging between 0.1 and 0.5 mm.

5. The medical implant (1) according to claim 1, **characterized in that** the primary coil (1') consists of a wire (2) having a diameter ranging between 0.03 and 0.3 mm and preferably between 0.05 and 0.2 mm.

6. The medical implant (1) according to any one of the above claims, **characterized in that** the coil (1') consists at least partially of a material having shape memory properties.

7. The medical implant (1) according to claim 6, **characterized in that** the material is a metallic alloy capable of passing through a stress-induced martensitic transformation.

8. The medical implant (1) according to claim 7, **characterized in that** the alloy is capable of passing through a temperature-induced martensitic transformation.

9. The medical implant (1) according to claim 6 or 7, **characterized in that** the alloy is an alloy containing titanium and nickel, an iron-based or copper-based alloy.

10. The medical implant (1) according to any one of the above claims, **characterized in that** the retaining element (14) is a metallic wire, preferably a wire consisting of medical stainless steel.

11. The medical implant (1) according to any one of the claims 1 to 10, **characterized in that** the pitches of the coil loops formed by the superimposed structure vary over the length of the implant (1).

12. The medical implant (1) according to claim 11, **characterized in that** the pitches reduce from the middle towards the ends.

13. The medical implant according to any one of the above claims, **characterized by** at least one severance module arranged therein provided with an electrolytically corrodible location.

14. The medical implant according to claim 13, **characterized in that** it is provided with severance modules arranged at regular intervals.

15. Device (7) for the placement of implants in body vessels and cavities with an implant (1) in accordance with any one of the above claims 1 to 14 and an insertion aid (10) which is detachably connected to the proximal end of the implant (1).

16. The device (7) according to claim 15, **characterized in that** the insertion aid (10) has the form of a tube, with the retaining element (14) extending through the lumen of such tube from the implant (1) in the proximal direction.

17. The device (7) according to claim 16, **characterized in that** the implant (1) and insertion aid (10) are interconnected by a severance module (8).

18. The device (7) according to any one of the claims 15 to 17 comprising a catheter, a voltage source and a cathode, with the implant (1) serving as anode and being longitudinally movable in the catheter, with the connection between implant (1) and insertion aid (10) having an electrolytically corrodible location (9) so that the implant (1) can be detached by electrolytic processes when in contact with a body fluid.

19. The device (7) according to claim 18, **characterized in that** the severance module (8) is provided with a proximal and a distal coil (4/4') as well as a segment (9) arranged in between, with the coils (4/4') consisting of a material whose susceptibility to electrolytic corrosion is lower than that of the interposed segment (9).

20. The device (7) according to claim 19, **characterized in that** the severance module (8) is non-detachably connected to the implant (1) and the insertion aid (10) by welding, soldering, bonding or mechanical joining processes, particularly by force- or form-closing methods.

21. The device (7) according to any one of the claims 15 to 20, **characterized in that** the insertion aid (10) has, at least in part, the form of a coil or spring.

22. The device (7) according to any one of the claims 15 to 20, **characterized in that** the insertion aid (10) is surrounded, at least in part, by an electrically insulating shrunk-on sleeve or an electrically insulating coating.

## Revendications

1. Implant médical de la forme d'au moins un enroulement primaire **(1')** oblong, l'enroulement primaire **(1')** étant préformé pour prendre une structure de niveau supérieur, qu'il adopte pendant l'implantation sur le site d'implantation, l'enroulement primaire **(1')** étant traversé sur son axe longitudinal par au moins un élément de maintien **(14)** qui peut être retiré de lui, **caractérisé en ce que** l'enroulement primaire **(1')** est empêché jusqu'à son retrait de prendre la structure de niveau supérieur et **en ce que** la structure de niveau supérieur est un enroulement secondaire.

2. Implant médical **(1)** selon la revendication 1, **caractérisé en ce que** la structure de niveau supérieur représente une forme essentiellement tubulaire.

3. Implant médical **(1)** selon la revendication 2, **caractérisé en ce que** l'enroulement secondaire présente un diamètre extérieur de 0,5 à 10 mm.

4. Implant médical (1) selon la revendication 1, **caractérisé en ce que** l'enroulement primaire (1') présente un diamètre extérieur de 0,1 à 0,5 mm.

5. Implant médical **(1)** selon la revendication 1, **caractérisé en ce que** l'enroulement primaire **(1')** est constitué d'un fil **(2)** d'un diamètre de 0,03 à 0,3 mm et de préférence compris entre 0,05 et 0,2 mm.

6. Implant médical **(1)** selon l'une des revendications précédentes, **caractérisé en ce que** l'enroulement **(1')** est composé au moins partiellement d'un matériau à propriétés de mémoire de forme.

7. Implant médical **(1)** selon la revendication 6, **caractérisé en ce que** le matériau est un alliage métallique ayant la capacité de connaître une transformation martensitique induite par des contraintes.

8. Implant médical **(1)** selon la revendication 7, **caractérisé en ce que** l'alliage présente la capacité de connaître une transformation martensitique induite par la température.

9. Implant médical **(1)** selon l'une des revendications 6 ou 7, **caractérisé en ce que** l'alliage est un alliage contenant du titane et du nickel, un alliage à base de fer ou un alliage à base de cuivre.

10. Implant médical **(1)** selon l'une des revendications précédentes, **caractérisé en ce que** l'élément de maintien **(14)** est un fil métallique, de préférence un fil en acier inoxydable à usage médical.

11. Implant médical **(1)** selon l'une des revendications 1 à 10, **caractérisé en ce que** les inclinaisons des boucles d'enroulement formées par la structure de niveau supérieur varient sur la longueur de l'implant **(1).**

12. Implant médical **(1)** selon la revendication 11, **caractérisé en ce que** les inclinaisons diminuent du centre vers les extrémités.

13. Implant médical selon l'une des revendications précédentes, **caractérisé par** au moins un module de détachement placé dedans, avec un endroit corrodable électrolytiquement.

14. Implant médical selon la revendication 13, **caractérisé en ce qu'**il présente des modules de détachement placés à intervalles réguliers.

15. Dispositif **(7)** pour l'implantation d'implants dans des vaisseaux corporels et des cavités avec un implant **(1)** selon l'une des revendications 1 à 14 ci-dessus et un accessoire d'introduction **(10)** qui est relié de manière amovible à l'extrémité proximale de l'implant **(1).**

16. Dispositif **(7)** selon la revendication 15, **caractérisé en ce que** l'accessoire d'introduction **(10)** est conformé en tube, à travers l'ouverture duquel l'élément de maintien **(14)** s'étend depuis l'implant **(1)** en direction du côté proximal.

17. Dispositif **(7)** selon la revendication 16, **caractérisé en ce que** l'implant **(1)** et l'accessoire d'introduction **(10)** sont reliés entre eux par un module de détachement **(8).**

18. Dispositif **(7)** selon l'une des revendications 15 à 17, avec un cathéter, une source de tension et une cathode, dans lequel l'implant **(1)** sert d'anode et est mobile dans la direction longitudinale dans le cathéter, la liaison entre l'implant **(1)** et l'accessoire d'introduction **(10)** présentant une zone **(9)** corrodable électrolytiquement, de sorte que l'implant **(1)** en contact avec un liquide corporel peut être détaché par des processus électrolytiques.

19. Dispositif **(7)** selon la revendication 18, **caractérisé en ce que** le module de détachement **(8)** comprend un enroulement proximal et un enroulement distal **(4/4')** ainsi qu'un segment **(9)** situé entre eux, les enroulements **(4/4')** étant constitués d'un matériau qui est moins sensible à la corrosion électrolytique que celui du segment intermédiaire **(9).**

20. Dispositif **(7)** selon la revendication 19, **caractérisé en ce que** le module de détachement **(8)** est relié de manière amovible à l'implant **(1)** resp. à l'accessoire d'introduction **(10)** par soudage, brasage, collage ou assemblage mécanique, en particulier par adhérence resp. par engagement positif.

21. Dispositif **(7)** selon l'une des revendications 15 à 20, **caractérisé en ce que** l'accessoire d'introduction **(10)** est réalisé au moins partiellement en enroulement ou en ressort.

22. Dispositif **(7)** selon l'une des revendications 15 à 20, **caractérisé en ce que** l'accessoire d'introduction **(10)** est entouré au moins partiellement d'une gaine rétrécissable isolante électrique ou d'un revêtement isolant électrique.
